# EUROPEAN PATENT SPECIFICATION

(11) **EP 1 608 420 B1**
(45) Date of publication and mention of the grant of the patent: **02.11.2006**
(21) Application number: 04724542.8
(22) Date of filing: 31.03.2004
(51) Int. Cl.: A61M 5/158, A61M 5/142, A61F 13/02

(54) **AN INFUSION DEVICE AND AN ADHESIVE SHEET MATERIAL AND A RELEASE LINER**
INFUSIONSVORRICHTUNG UND KLEBEFOLIENMATERIAL UND ABZIEHSCHICHT
DISPOSITIF DE PERFUSION, MATIÈRE DE FEUILLE ADHESIVE ET COUCHE DE PROTECTION

(30) Priority: 01.04.2003 DK 200300495; 01.04.2003 US 404340
(43) Date of publication of application: 28.12.2005
(73) Proprietor: Unomedical A/S, 3460 Birkerød (DK)
(72) Inventor: NIELSEN, Jens, Egebjerg, DK-4100 Ringsted (DK)
(74) Representative: Sundien, Thomas
(86) International application number: PCT/DK2004/000221
(87) International publication number: WO 2004/087240

(56) References cited:
- EP-A- 0 239 244
- WO-A-95/28327
- DE-A- 3 715 965
- US-A- 6 045 533

## Description

The invention relates to an infusion device comprising a housing with an upper face plate and a lower face plate and a cannula connected to the lower face plate and through which a liquid is injectable into the skin of a user; means for injecting the liquid and an adhesive sheet material placed in connection with the lower face plate and for securing the infusion device to the skin, said adhesive sheet material comprising a backing layer which has, on a portion of one surface, an adhesive layer, said adhesive layer being covered by a removable release liner, said sheet and release liner comprising a central aperture through which the cannula may act.

The invention also relates to an adhesive sheet material comprising a backing layer which has, on a portion of one surface, an adhesive layer, wherein the adhesive layer is covered by a removable release liner, said sheet and release liner comprising a central aperture.

Additionally, the invention relates to a release liner for covering an adhesive sheet material, said adhesive sheet material comprising a backing layer which has, on a portion of one surface, an adhesive layer, said adhesive layer being covered by the removable release liner

International application No WO 03/026728 teaches an injection device for being located on the skin of a user, and from where eg insulin is injected via a cannula into the user, a subcutaneous infusion set being located on the skin by means of the injection device. The infusion device can be adhered to the skin by means of an adhesive pad that is connected to the lower face of the infusion device and, with its adhesive face, faces towards the skin of the user. Moreover, centrally of the pad/the adhesive sheet a recess will be provided corresponding to the cannula and a needle, located either here on upon activation emerging unimpededly. Upon penetration of the skin and subsequent activation of means in the injection device, injection is performed of the substance in question, which is preferably insulin.

Prior to arrangement of the infusion device on the skin it is required that the release liner arranged on the adhesive face of the pad is removed. Typically, this is accomplished either by the release liner having several score lines for removing the release liner, or it is an option to remove it in one circular piece which is, however, difficult, without an ensuing risk of soiling the adhesive face and, likewise, it may be difficult to handle the injection device and hence there is a risk of losing the product.

From EP-0-239-244 such an injection device with an adhesive sheet material is known, the adhesive sheet comprising a backing layer, an adhesive layer and a removable release liner, said sheet and release liner comprising a central aperture through which the cannula may act, said release liner comprising at a score line constituting a straight line starting at the periphery of the release liner and ending on the border of the periphery of the release liner's central aperture.

Furthermore, an adhesive sheet is known from US 5,384,174, that teaches a circular pad for being located on a user's skin, and wherein an adhesive face is arranged behind a release liner. The release liner is removed prior to application of the pad on the skin, as it typically comprises a pair of score lines in the release liner, such that the liner is removed in two, three pieces. There is consequently a risk that, during removal of this release liner, the user accidentally touches the adhesive face thereby transferring sebum particles to this, thereby reducing its ability to adhere to the skin. Likewise, the shape of the release liner is a contributing factor why it is difficult to handle, and therefore there is a risk of loosing it during handling of the pad.

From WO 00/44324 an adhesive pad being covered by a removable release liner having one or more score lines is known wherein the pad and release liner has a central aperture, and the where the score lines extends as straight lines starting at the periphery of the release liner and ending on the border of the periphery of the release liner's central aperture.

The above mentioned liners all have the disadvantage that when they are removed the removal necessarily needs to be conducted very carefully with a movement encircling the aperture in the adhesive pad or sheet material to avoid tearing of the liner material. Especially if it is attempted to remove the liner by a straight upwards or sideways pull - which is the easiest and most instinctive move of the hand in this situation - the risk of tearing the liner from the inner periphery of the central aperture and outward is imminent. In the situations where such liners need to be removed i.e. during the placing of an injection device or adhesive pad the attention of the user is directed towards the placing of the device or pad and not towards a specific movement of the hand during the removal of a liner. This tearing in itself may reduce the adhesive capabilities of the adhesive sheet material, by -the shear to this material creating kinks in the adhesive pad or tearing the adhesive material fro the injection device. If the liner is torn pieces of the liner may stick to the adhesive sheet, which needs to be removed. This in itself, is a disadvantage because it will prolong the act of placing the injection device or adhesive pad. Further, when removing the pieces by hand sebum particles are easily transferred to the adhesive face thereby decreasing the adhesive capabilities of the adhesive face. Also there is a risk of contamination of the adhesive face, which is highly undesirable since these faces are placed near perforations or lesions to the skin of a patient thus risking infections. Further in the case of injection devices the wrongful removal of the liner may cause unintentional hits to the cannula or needle. Also in this case handling pieces stuck on the adhesive face in the vicinity of the needle or cannula the user risks being stuck by the needle, or the needle can be damaged or contaminated.

It is thus the object of the present invention to remedy the above-identified problems and to provide a release liner or an adhesive material covered by a release liner and that can be used either alone or in combination with an infusion device, said release liner being easily removable in one piece as a strip, and in a single upwardly directed straight movement of the hand, thereby reducing the risk of the user handling the product erroneously, and, likewise, little force is needed to remove the release liner since, other things being equal, all it takes is just to overcome the adhesive force of the piece of strip currently being removed by the user.

This object is obtained with an infusion device as featured in the preamble to claim 1 and wherein the score line constitutes a spiral curve centered around the aperture (105).

This object is also achieved by an adhesive sheet as taught in the preamble to claim 5 and wherein the score line constitutes a spiral curve centered around the aperture (105).

This object is also achieved by a release liner as taught in the preamble to claim 9 and wherein the score line constitutes a spiral curve centered around the aperture (105).

Thus, the shear forces to the sides - particularly to the side facing the central aperture of the adhesive sheet - during removal of the liner is reduced because the forces acting on the liner are not directed generally perpendicularly to the side of the liner strip, but rather generally parallel thereto.

Thus also a convenient shape of the score line is accomplished since a helical scoring brings about a simple and reproducible strip; albeit it is an option that the helical score line thus provided could bend and assume such course that the distance from the sheet periphery to the score line was not continuously decreasing, but rather decreasing, increasing, decreasing, and so on, however avoiding drastic kinks in the curve.

Thus the invention works in that, once the pad or an adhesive substance covered by a release liner is located on the infusion device, the user seizes the injection device and with the adhesive face facing towards him, he seizes a portion of the release liner, where a starting area will primarily be a tab. The tab will be situated outside the delimitation of the pad as such or the glue and it is used as starting point for stripping off the release liner in one piece. The shape of the adhesive sheet is preferably circular. The sheet may also be configured with other shapes, including oval, triangular, rectangular, etc., and wherein the scoring is performed helically so as to take its starting point in the periphery of the release liner and coil inwardly in a single helix, where it ends at a central opening where the cannula either protrudes or will be caused to protrude upon activation.

As mentioned, by a single straight pull the release paper can be removed in one single narrow strip and this is why only little force is needed to remove it, the handling is easy and, likewise, tearing of the liner can be avoided. The handling will take place in the same manner as in case the adhesive sheet were used without an injection device, eg as an ordinary band aid. It is thus a crucial point that the scoring that takes place brings about a strip and that the score line thus generated does not at any point intersect itself as this would result in a number of separate sheet papers.

Now, the injector device is subsequently activated, whereby the infusion device is located on the user's skin and, via the adhesive face, its contact therewith is hereby ensured; for further reference see also international application No WO 03/026728.

By providing an infusion device as featured in claim 2 and an adhesive sheet material as featured in claim 6 and a release line as featured in claim 10, an especially advantages embodiment of the invention, where the shear forces acting on the side of liner is reduced to a minimum and consequently the risk of tearing the liner during removal is obtained

By providing an infusion device as featured in claim 3 and an adhesive sheet material as featured in claim 7 and a release line as featured in claim 11, a much used shape of the adhesive sheet and the release liner is obtained.

By providing an infusion device according to the invention and as further featured in claim 4, and an adhesive sheet according to the invention and as further featured in claim 8, and a release liner according to the invention, and as further featured in claim 12, a good starting area is obtained for stripping off the release liner.

The invention will now be explained in further detail with reference to the drawing, wherein:
Figure 1 shows an injection device in a sectional view and as known from international application No WO 03/026728, on which an adhesive sheet is arranged and comprising a release liner according to the invention;
Figure 2 shows an infusion device suitable for use with an injector device disclosed in fig. 1;
Figure 3a shows adhesive sheet material, in a side view;
Figure 3b shows an adhesive sheet material, seen from the bottom, and wherein the release liner with helical score line applied;
Figure 4 shows a rectangular adhesive sheet material with a helical score line for providing a removable release liner;
Figure 5 shows an oval adhesive sheet material with a helical score line for providing a removable release liner;
Figure 6 shows an adhesive sheet material shaped to be essentially circular and having an applied release liner comprising a score line that is an unbroken line that extends curved and in a helix that coils towards the centre to the central opening;
in principle Figure 7 shows the same as Figure 6, but wherein the design is a rectangular sheet material.

### DETAILED DESCRIPTION OF THE PREFERRED EMBODIMENTS

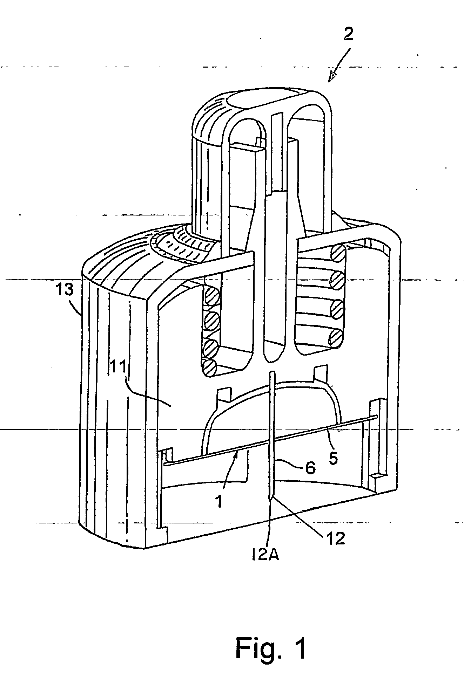

An injector device shown schematically in fig. 1 by the reference numeral 2 is provided for quick and easy placement of a subcutaneous infusion set 1, and may then be discarded safely. The infusion set 1 with a cannula 6 extending there from is shown schematically only.

The injector device 2 includes a plunger 11 having thereon a medical insertion needle 12 with a pointed end 12A. The plunger 11 is arranged for longitudinal sliding movement within a device housing 13 between a forward advanced position and a rearward retracted position. The device housing 13 may have a circular, square or any desired cross-sectional shape. The device housing 13 and the plunger 11 are preferably formed of a plastics material in a moulding process.

The infusion set 1 is used to infuse medical fluids such as insulin to a patient, and generally includes a housing with an internal chamber (not shown) that receives medication via infusion tubing. An enlarged base of the infusion set 1, also designated a lower face plate 5, is provided on the housing for stable affixation thereof to the skin of the patient. The enlarged base 5 carries an adhesive and is provided with a release sheet or removable release liner 104 which is removed to expose the adhesive prior to placement of the infusion set. Alternatively, the base 5 may be sized to allow the infusion device to be fixed to the patient by an adhesive patch. The infusion set has a protruding soft and flexible cannula 6, which communicates with the internal chamber, and a passage sealed by a sealing membrane extends through the housing opposite the cannula 6. The medical insertion needle 12 of the injector device 2 extends through the passage, into the internal chamber and through the cannula 6, when the infusion set 1 is mounted in position on the injector device. After transcutaneous placement of the cannula 6, the injector device 2 with the insertion needle 12 is retracted from the infusion set 1 to permit medication delivery through the cannula 6 to the patient.

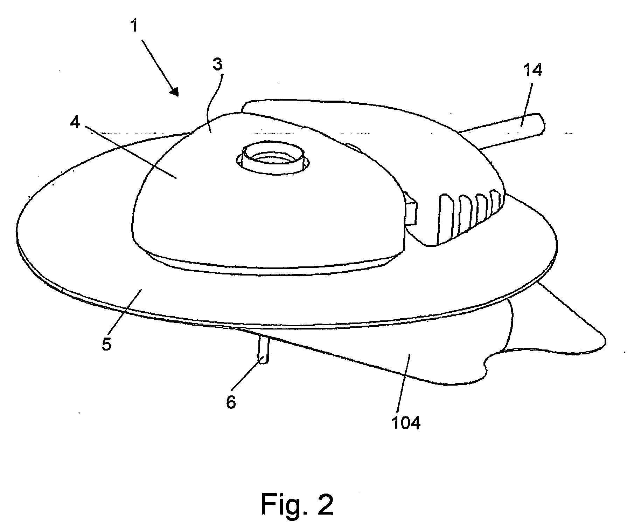

Fig. 2 shows an example of an infusion set 1 suitable for use with an injector device. The infusion set 1 includes a housing 3 with an internal chamber (not shown). The internal chamber receives medication via infusion tubing 14 which may be detachably connected to the housing 3 by any suitable connector. The medication is delivered by means for injecting the liquid for instance a pump or syringe. The base 5 of the housing 3 and opposite the upper face, plate 4 may carry an adhesive and be provided with a release sheet 104 which is removed to expose the adhesive prior to placement of the infusion set. The infusion set 1 has a protruding soft and flexible cannula 6, which communicates with the internal chamber. An internal passage which is sealed by a sealing membrane and which is penetrated by the insertion needle of the injector device extends through the housing opposite the cannula 6.

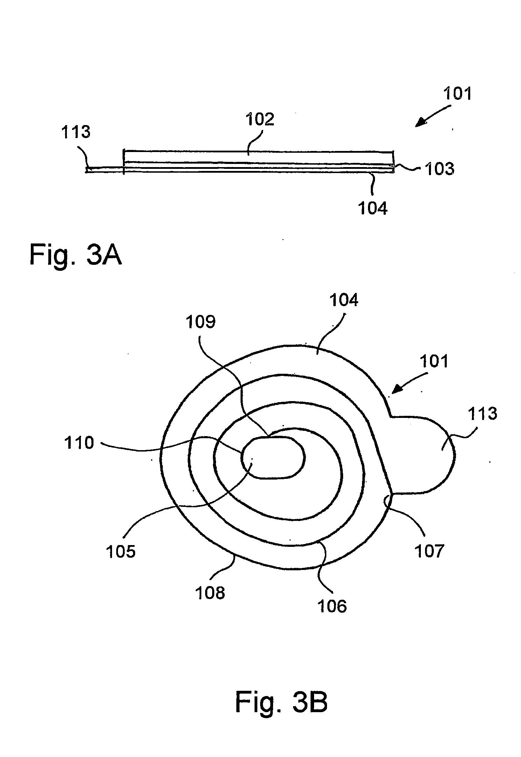

Figure 3a shows an adhesive sheet material 101 that is suitable for being arranged on the lower face plate 5 shown in Figure 2. The adhesive sheet material 101 comprises a backing layer 102 that can be manufactured from polyester fibre and on the one face of which an adhesive layer 103 is applied that is conveniently covered with a release liner 104, said release liner being a non-adhesive paper, which distinguishes itself in that, following removal thereof from adhesive layer, the adhesive remains on the backing layer, and, likewise, no residue of the adhesive substance remains on the release liner as such.

Figure 3b shows a preferred embodiment comprising an essentially circular sheet material, at the centre of which a central aperture 105 is provided that can be configured to be oval, circular, triangular and in which, in connection with an infusion device, provides space for the cannula. This aperture is delimited by a border line 110. The outer delimitation of the removable release liner is referred to as the periphery 108. Optionally, this periphery may comprise a tab 113, which is preferably constituted exclusively by the release liner and will thus serve as starting area for stripping off the release liner. In the periphery 108 of the release liner is a starting point 107 for the score line 106, said score line 106 extending helically and in an unbroken line towards the centre of the release liner and ending in the edge of the central aperture 105 in an end point 109. Thus, if the tag/tab 113 is seized, it will require only little force to remove the liner from the subjacent adhesive layer, and the release liner can be stripped off in one single strip, leaving the adhesive face 103 exposed.

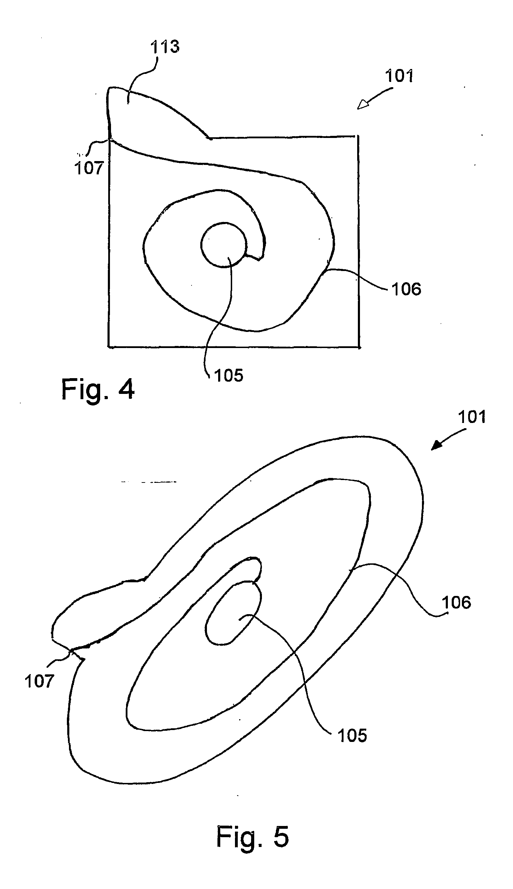

Figure 4 shows an example of a rectangular sheet material and a rectangular release liner, and wherein a score line 106 is provided that also extends helically and with a starting point 107 peripherally of the adhesive sheet material and ending at the centre where the central aperture 105 is provided.

Figure 5 shows a corresponding system wherein the pad with release liner has an oval shape.

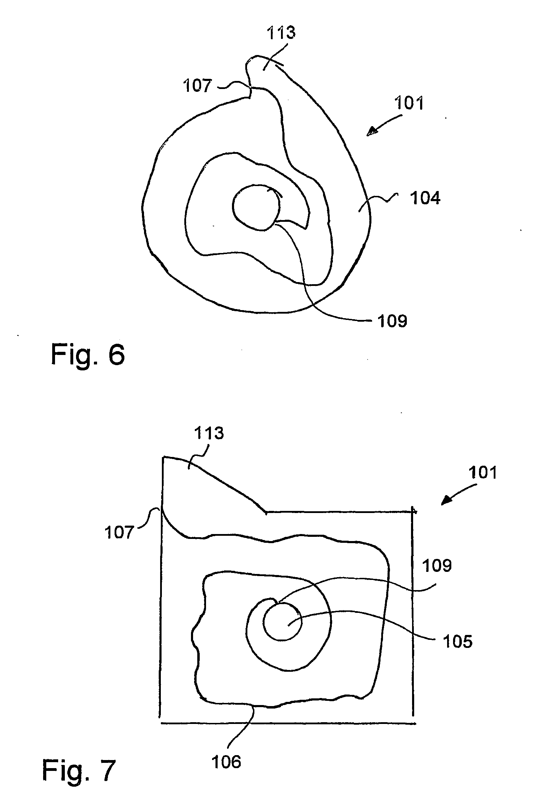

Figure 6 shows essentially the same as Figure 3b, but wherein the score line 106 does not have an evenly decreasing distance to the outer periphery 108, but rather extends in a curved manner such that the distance to the outer periphery 108 increases/decreases/increases, and wherein the essential aspect is that the score line 106 does not at any point intersect itself, since this would mean that the removable release liner 104 cannot be removed in one piece as it is one of the objects of the present invention. Therefore it is important that no kinks in the curve appear. Even though the distance to the central aperture 105 may locally increase again over the course of the curve as seen from the start 107 to the end 109, the general shape of the curve needs to be spiraling inward.

Finally in principle Figure 7 shows the same as Figure 4, but wherein a more curved course is provided and wherein the score line is not shaped circularly, but has a rather rectangular course.

## Claims

1. An infusion device (1) comprising a housing (3) with an upper face plate (4) and a lower face plate (5) and a cannula (6) connected to the lower face plate (5) and through which a liquid is injectable into the skin of a user; means for injecting the liquid, and an adhesive sheet material (101) placed in connection with the lower face plate (5) and for securing the infusion device (1) to the skin; said adhesive sheet material (101) comprising a backing layer (102) which has, on a portion of one surface, an adhesive layer (103); said adhesive layer (103) being covered by a removable release liner (104), said sheet and release liner (104) comprising a central aperture (105) through which the-cannula (6) may act, said release-liner (104) comprising at least one score line (106) for stripping the release liner (104) of the adhesive layer (103), said score line (106) having a starting point (107) placed in the periphery (108) of the release liner (104), said score line (106) being an unbroken line that does not intersect itself and continues to an end point (109) placed on the border of the periphery (110) of the release liner's central aperture (105), **characterized in that** the score line (106) constitutes a spiral curve centered around the aperture (105).

2. An infusion device (1) according to claim 1, **characterized in that** the score line (106) evolves at least on time around the central aperture (105).

3. An infusion device (1) according to claim 1 or 2, **characterized in that** the adhesive sheet (101) and release liner (104) are substantially circular

4. An infusion device (1) according to any one of claims 1-3, **characterized in that** the release liner (104) comprises a tab (113) for gripping the release liner (104) and starts the stripping from the starting point (107).

5. An adhesive sheet material (101) comprising a backing layer (102) which has, on a portion of one surface, an adhesive layer (103), said adhesive layer (103) being covered by a removable release liner (104), said sheet and release liner (104) comprising a central aperture (105), said release liner (104) comprising at least one score line (106) for stripping the release liner (104) of the adhesive layer (103), said score line (106) having a starling point (107) placed in the periphery (108) of the release liner (104), said score line (106) being an unbroken line that does not intersect itself and continues to an end point (109) placed on the border of the periphery (110) of the release liner's central aperture (105), **characterized in that** the score line (106) constitutes a spiral curve centered around the aperture (105).

6. An adhesive sheet material (101) according to claims 5, **characterized in that** the score line (106) evolves at least on time around the central aperture (105).

7. An adhesive sheet material (101) according to claim 5 or 6, **characterized in that** the adhesive (101) sheet and the release liner (104) are substantially circular.

8. An adhesive sheet material (101) according to any one of claims 5-7, **characterized in that** the release liner (104) comprises a tab (113) for gripping the release liner (104) and starts the stripping from the starting point (107).

9. A release liner (104) for covering an adhesive sheet material (101), wherein said adhesive sheet material (101) comprises a backing layer (102) which has, on a portion of one surface, an adhesive layer (103), said adhesive layer (103) being covered by the removable release liner (104), said sheet and release liner (104) comprising a central aperture (105), said release liner (104) comprising at least one score line (106) for stripping the release liner (104) of the adhesive layer (103), said score line (106) having a starting point (107) placed in the periphery (108) of the release liner (104), said score line (106) being an unbroken line that does not intersect itself and continues to an end point (109) placed on the border of the periphery (110) of the release liner's central aperture (105), **characterised in that** the score line (106) constitutes a spiral curve centered around the aperture (105).

10. A release liner (104) according to claim 9, **characterized in that** the score line (106) evolves at least on time around the central aperture (105).

11. A release liner (104) according to claim 9 or 10, **characterized in that** the release liner (104) is substantially circular.

12. A release liner according to any one of claims 9-11, **characterized in that** the release liner (104) comprises a tab (113) for gripping the release liner (104) and starts the stripping from the starting point (107).

## Patentansprüche

1. Infusionsvorrichtung (1), umfassend ein Gehäuse (3) mit einer oberseitigen Platte (4) und einer unterseitigen Platte (5) und einer Kanüle (6), die mit der unterseitigen Platte (5) verbunden ist und durch welche eine Flüssigkeit in die Haut eines Benutzers injizierbar ist; Mittel zum Injizieren der Flüssigkeit, und ein Klebefolienmaterial (101), das in Verbindung mit der unterseitigen Platte (5) platziert ist und zum Sichern der Infusionsvorrichtung (1) an der Haut dient; wobei das Klebefolienmaterial (101) eine Rückschicht (102) aufweist, welche, auf einem Abschnitt einer Oberfläche, eine Klebeschicht (103) besitzt; wobei die Klebeschicht (103) von einer entfernbaren Abziehschicht (104) abgedeckt ist, wobei die Folie und die Abziehschicht (104) eine mittige Öffnung (105) enthalten, durch welche die Kanüle (6) ihre Funktion ausüben kann, wobei die Abziehschicht (104) mindestens eine Schwächungslinie (106) zum Abziehen der Abziehschicht (104) von der Klebeschicht (103) aufweist, wobei die Schwächungslinie (106) einen Anfangspunkt (107) hat, der in der Peripherie (108) der Abziehschicht (104) platziert ist, wobei die Schwächungslinie (106) eine ununterbrochene Linie ist, die sich nicht selbst schneidet und sich bis zu einem Endpunkt (109) fortsetzt, der am Rand der Peripherie (110) der mittigen Öffnung (105) der Abziehschicht platziert ist, **dadurch gekennzeichnet, dass** die Schwächungslinie (106) eine spiralförmige Windung mit der Öffnung (105) als Mittelpunkt bildet.

2. Infusionsvorrichtung (1) nach Anspruch 1, **dadurch gekennzeichnet, dass** die Schwächungslinie (106) sich zumindest rechtzeitig um die mittige Öffnung (105) windet.

3. Infusionsvorrichtung (1) nach Anspruch 1 oder 2, **dadurch gekennzeichnet, dass** die Klebefolie (101) und die Abziehschicht (104) im Wesentlichen kreisförmig sind.

4. Infusionsvorrichtung (1) nach einem der Ansprüche 1-3, **dadurch gekennzeichnet, dass** die Abziehschicht (104) eine Lasche (113) zum Greifen der Abziehschicht (104) aufweist und das Abziehen vom Anfangspunkt (107) aus beginnt.

5. Klebefolienmaterial (101), umfassend eine Rückschicht (102), welche, auf einem Abschnitt einer Oberfläche, eine Klebeschicht (103) aufweist, wobei die Klebeschicht (103) von einer entfernbaren Abziehschicht (104) abgedeckt ist, wobei die Folie und die Abziehschicht (104) eine mittige Öffnung (105) enthalten, wobei die Abziehschicht (104) mindestens eine Schwächungslinie (106) zum Abziehen der Abziehschicht (104) von der Klebeschicht (103) aufweist, wobei die Schwächungslinie (106) einen Anfangspunkt (107) hat, der in der Peripherie (108) der Abziehschicht (104) platziert ist, wobei die Schwächungslinie (106) eine ununterbrochene Linie ist, die sich nicht selbst schneidet und sich bis zu einem Endpunkt (109) fortsetzt, der am Rand der Peripherie (110) der mittigen Öffnung (105) der Abziehschicht platziert ist, **dadurch gekennzeichnet, dass** die Schwächungslinie (106) eine spiralförmige Windung mit der Öffnung (105) als Mittelpunkt bildet.

6. Klebefolienmaterial (101) nach Anspruch 5, **dadurch gekennzeichnet, dass** die Schwächungslinie (106) sich zumindest rechtzeitig um die mittige Öffnung (105) windet.

7. Klebefolienmaterial (101) nach Anspruch 5 oder 6, **dadurch gekennzeichnet, dass** die Klebefolie (101) und die Abziehschicht (104) im Wesentlichen kreisförmig sind.

8. Klebefolienmaterial (101) nach einem der Ansprüche 5-7, **dadurch gekennzeichnet, dass** die Abziehschicht (104) eine Lasche (113) zum Greifen der Abziehschicht (104) aufweist und das Abziehen vom Anfangspunkt (107) aus beginnt.

9. Abziehschicht (104) zum Abdecken eines Klebefolienmaterials (101), wobei das Klebefolienmaterial (101) eine Rückschicht (102) aufweist, welche, auf einem Abschnitt einer Oberfläche, eine Klebeschicht (103) aufweist, wobei die Klebeschicht (103) von der entfernbaren Abziehschicht (104) abgedeckt ist, wobei die Folie und die Abziehschicht (104) eine mittige Öffnung (105) enthalten, wobei die Abziehschicht (104) mindestens eine Schwächungslinie (106) zum Abziehen der Abziehschicht (104) von der Klebeschicht (103) aufweist, wobei die Schwächungslinie (106) einen Anfangspunkt (107) hat, der in der Peripherie (108) der Abziehschicht (104) platziert ist, wobei die Schwächungslinie (106) eine ununterbrochene Linie ist, die sich nicht selbst schneidet und sich bis zu einem Endpunkt (109) fortsetzt, der am Rand der Peripherie (110) der mittigen Öffnung (105) der Abziehschicht platziert ist, **dadurch gekennzeichnet, dass** die Schwächungslinie (106) eine spiralförmige Windung mit der Öffnung (105) als Mittelpunkt bildet.

10. Abziehschicht (104) nach Anspruch 9, **dadurch gekennzeichnet, dass** die Schwächungslinie (106) sich zumindest rechtzeitig um die mittige Öffnung (105) windet.

11. Abziehschicht (104) nach Anspruch 9 oder 10, **dadurch gekennzeichnet, dass** die Abziehschicht (104) im Wesentlichen kreisförmig ist.

12. Abziehschicht nach einem der Ansprüche 9-11, **dadurch gekennzeichnet, dass** die Abziehschicht (104) eine Lasche (113) zum Greifen der Abziehschicht (104) aufweist und das Abziehen vom Anfangspunkt (107) aus beginnt.

## Revendications

1. Dispositif de perfusion (1) comprenant un boîtier (3) avec une plaque de face supérieure (4) et une plaque de face inférieure (5) et une cannule (6) reliée à la plaque de face inférieure (5) et à travers laquelle un liquide peut être injecté dans la peau d'un utilisateur; un moyen pour injecter le liquide, et un matériau de feuille adhésive (101) placé en rapport avec la plaque de face inférieure (5) et pour attacher le dispositif de perfusion (1) à la peau; ledit matériau de feuille adhésive (101) comprenant une couche de support (102) qui présente, sur une portion d'une surface, une couche adhésive (103); ladite couche adhésive (103) étant couverte par un revêtement de séparation amovible (104), ladite feuille et le revêtement de séparation (104) comprenant une ouverture centrale (105) à travers laquelle la cannule (6) peut agir, ledit revêtement de séparation (104) comprenant au moins une ligne de repère (106) pour retirer le revêtement de séparation (104) de la couche adhésive (103), ladite ligne de repère (106) ayant un point de départ (107) placé dans la périphérie (108) du revêtement de séparation (104), ladite ligne de repère (106) étant une ligne non interrompue qui ne se croise pas avec elle-même et qui continue à un point final (109) placé sur le bord de la périphérie (110) de l'ouverture centrale du revêtement de séparation (105), **caractérisé en ce que** la ligne de repère (106) constitue une courbe en spirale centrée autour de l'ouverture (105).

2. Dispositif de perfusion (1) selon la revendication 1, **caractérisé en ce que** la ligne de repère (106) évolue au moins une fois autour de l'ouverture centrale (105).

3. Dispositif de perfusion (1) selon la revendication 1 ou 2, **caractérisé en ce que** la feuille adhésive (101) et le revêtement de séparation (104) sont sensiblement circulaires.

4. Dispositif de perfusion (1) selon l'une des revendications 1 à 3, **caractérisé en ce que** le revêtement de séparation (104) comprend une patte (113) pour saisir le revêtement de séparation (104) et commence l'enlèvement à partir du point de départ (107).

5. Matériau de feuille adhésive (101) comprenant une couche de support (102) qui présente, sur une portion d'une surface, une couche adhésive (103), ladite couche adhésive (103) étant recouverte d'un revêtement de séparation amovible (104), ladite feuille et le revêtement de séparation (104) comprenant une ouverture centrale (105), ledit revêtement de séparation (104) comprenant au moins une ligne de repère (106) pour retirer le revêtement de séparation (104) de la couche adhésive (103), ladite ligne de repère (106) ayant un point de départ (107) placé dans la périphérie (108) du revêtement de séparation (104), ladite ligne de repère (106) étant une ligne non interrompue qui ne se croise pas avec elle-même et qui continue à un point final (109) placé sur le bord de la périphérie (110) de l'ouverture centrale du revêtement de séparation (105), **caractérisé en ce que** la ligne de repère (106) forme une courbe en spirale centrée autour de l'ouverture (105).

6. Matériau de feuille adhésive (101) selon la revendication 5, **caractérisé en ce que** la ligne de repère (106) évolue au moins une fois autour de l'ouverture centrale (105).

7. Matériau de feuille adhésive (101) selon la revendication 5 ou 6, **caractérisé en ce que** la feuille adhésive (101) et le revêtement de séparation (104) sont sensiblement circulaires.

8. Matériau de feuille adhésive (101) selon l'une des revendications 5 à 7, **caractérisé en ce que** le revêtement de séparation (104) comprend une patte (113) pour saisir le revêtement de séparation (104) et commence l'enlèvement au point de départ (107).

9. Revêtement de séparation (104) pour couvrir un matériau de feuille adhésive (101), où ledit matériau de feuille adhésive (101) comprend une couche de support (102) qui présente, sur une portion d'une surface, une couche adhésive (103), ladite couche adhésive (103) étant couverte par le revêtement de séparation amovible (104), ladite feuille et le revêtement de séparation (104) comprenant une ouverture centrale (105), ledit revêtement de séparation (104) comprenant au moins une ligne de repère (106) pour retirer le revêtement de séparation (104) de la couche adhésive (103), ladite ligne de repère (106) ayant un point de départ (107) placé dans la périphérie (108) du revêtement de séparation (104), ladite ligne de repère (106) étant une ligne non interrompue qui ne se croise pas avec elle-même et qui continue à un point final (109) placé sur le bord de la périphérie (110) de l'ouverture centrale (105) du revêtement de séparation, **caractérisé en ce que** la ligne de repère (106) forme une courbe en spirale centrée autour de l'ouverture (105).

10. Revêtement de séparation (104) selon la revendication 9, **caractérisé en ce que** la ligne de repère (106) évolue au moins une fois autour de l'ouverture centrale (105).

11. Revêtement de séparation (104) selon la revendication 9 ou 10, **caractérisé en ce que** le revêtement de séparation (104) est sensiblement circulaire.

12. Revêtement de séparation selon l'une des revendications 9 à 11, **caractérisé en ce que** le revêtement de séparation (104) comprend une patte (113) pour saisir le revêtement de séparation (104) et commence l'enlèvement au point de départ (107).
